# EUROPÄISCHE PATENTSCHRIFT

(11) **EP 1 881 322 B1**
(45) Veröffentlichungstag und Bekanntmachung des Hinweises auf die Patenterteilung: **15.06.2011**
(21) Anmeldenummer: 06117422.3
(22) Anmeldetag: 18.07.2006
(51) Int. Cl.: G01N 33/487, A61B 5/00

(54) **Bauraumoptimiertes tragbares Messsystem**
Space-optimised portable measuring system
Système portable de mesure, à optimisation spatiale

(43) Veröffentlichungstag der Anmeldung: 23.01.2008
(73) Patentinhaber: Roche Diagnostics GmbH, 68305 Mannheim (DE); F. Hoffmann-La Roche AG, 4070 Basel (CH)
(72) Erfinder: HOENES, Joachim, D-64673 Zwingenberg (DE)
(74) Vertreter: Stößel, Matthias

(56) Entgegenhaltungen:
- EP-A- 1 637 078
- EP-A2- 1 203 563
- DE-A1- 10 332 488
- US-A1- 2004 092 842
- US-A1- 2005 033 196

## Beschreibung

### Gebiet der Erfindung

Die Erfindung betrifft ein tragbares Messsystem mit kleinem Bauraum, welches eingesetzt werden kann zur Analyse einer flüssigen Probe auf mindestens einen darin enthaltenen Analyten. Derartige Messsysteme werden insbesondere im Bereich der Medizintechnik, beispielsweise bei der Blutglukose-Überwachung, oder im Bereich der chemischen oder biologischen Analytik, beispielsweise der Umweltanalytik, eingesetzt.

### Stand der Technik

Die Überwachung der Blutglukosekonzentration ist für Diabetiker ein essentieller Bestandteil des Tagesablaufs. Dabei muss die Blutglukosekonzentration in der Regel schnell und zuverlässig mehrmals am Tag bestimmt werden, um gegebenenfalls entsprechende medizinische Maßnahmen ergreifen zu können. Um den Tagesablauf des Diabetikers nicht mehr als nötig einzuschränken, werden dabei häufig entsprechende mobile Geräte eingesetzt, welche einfach zu transportieren und zu handhaben sein sollten, so dass die Messung der Blutglukosekonzentration beispielsweise am Arbeitsplatz oder auch in der Freizeit erfolgen kann.

Derzeit sind verschiedene mobile Geräte auf dem Markt, welche teilweise nach unterschiedlichen Messmethoden funktionieren. Es kommen verschiedene Diagnoseverfahren zum Einsatz, beispielsweise optische oder auch elektrochemische Messverfahren. Ein Beispiel eines häufig eingesetzten Messverfahrens nutzt eine spezielle Art elektrochemischer Teststreifen, welche beispielsweise derart aufgebaut sind, dass eine vorgegebene Blutmenge über ein Kapillarensystem auf dem Teststreifen zu einem Elektrodensystem geführt wird. Für moderne Teststreifen genügt dabei eine Blutmenge von ca. 1,5 µl, teilweise auch Blutmengen unter 1 µl. Bei dem Elektrodensystem kann es sich z. B. um Goldelektroden handeln, welche mit einer Beschichtung versehen sind. Die Beschichtung enthält zumeist verschiedene Enzyme und so genannte Mediatoren und bewirkt, dass sich innerhalb der Probe an den Elektroden Ladungsträger (beispielsweise in Form von Redox-Molekülen) bilden, deren Konzentration abhängig ist von der Blutglukosekonzentration. Die Konzentration dieser Ladungsträger kann mittels der Goldelektroden und einem geeigneten Messsystem, beispielsweise mittels einer Strom-Spannungs-Messung, bestimmt werden, so dass daraus schließlich auf die Blutglukosekonzentration zurückgerechnet werden kann. Ein Beispiel derartiger elektrochemischer Teststreifen ist in US 5,286,362 dargestellt.

Alternativ zu dem beschriebenen elektrochemischen Messverfahren lassen sich auch andere Messprinzipien einsetzen. So beschreibt beispielsweise die WO 01/48461 einen Lichtleiter-Teststreifen zur Untersuchung einer Probe, insbesondere einer Körperflüssigkeit, bei dem ein Reagenzsystem bei Reaktion mit der Probe zu einer charakteristischen, optisch messbaren Änderung in einer Detektionszone führt. Über Lichtleiter, die in den Teststreifen eingelassen sind, kann diese Änderung von einem Auswertegerät ausgewertet werden.

Ein Messsystem für einen einzelnen Teststreifen wird in US 2005/033196 offenbart.

Die Teststreifen bilden somit ein wesentliches Element portabler Diagnosesysteme. Typischerweise werden von einem Diabetiker ca. 5 bis 7 derartiger Teststreifen pro Tag benötigt. Essentiell ist dabei, dass die Teststreifen sauber und trocken aufbewahrt werden, um nicht durch eine entsprechende Verschmutzung beziehungsweise Einwirkung von Feuchtigkeit die Messung der Blutglukosekonzentration zu verfälschen.

Zu diesem Zweck werden die Teststreifen üblicherweise in entsprechenden Behältnissen aufbewahrt, um anschließend vom Benutzer für eine Messung dem Teststreifenbehältnis entnommen und in ein entsprechendes Messgerät eingegeben zu werden. Derartige Messgeräte, beispielsweise Messgeräte für eine elektrochemische oder optische Bestimmung der Blutglukosekonzentration, sind dem Fachmann bekannt und werden beispielsweise in US 2002/0170823 A1 beschrieben.

Zum Aufbewahren und Ausgeben der Teststreifen sind auch Magazinsysteme bekannt. So beschreiben beispielsweise US 2003/0116583 A1, EP 0 640 393 B1 und US 4,911,344 entsprechende Aufbewahrungssysteme, in welchen mehrere Teststreifen in einem Magazin gelagert sind. EP 1 488 736 A1 beschreibt darüber hinaus ein System, in welchem anstelle einzelner Teststreifen eine Bandkassette eines einzelnen langen Teststreifens mit mehreren Testfeldern aufgenommen ist. Ein ähnliches System mit Bandkassette wird in DE 10332488 offenbart.

Neben Systemen, bei denen Teststreifenmagazin und Messgerät als getrennte Einheiten eingesetzt werden, existieren auch integrierte Systeme, bei welchen mehrere Teststreifen nicht nur in einem Magazin gelagert werden, sondern welche gleichzeitig auch die Möglichkeit einer Auswertung dieser Teststreifen bieten. Beispiele derartiger integrierter Systeme sind in US 5,489,414, US 6,093,156, WO 02/18940 A2, WO 02/055008 oder WO 03/083469 A2 zu finden. Teilweise beinhalten diese Systeme, wie beispielsweise das in US 6,093,156 oder WO 03/083469 A2 beschriebene System, auch bereits ein integriertes Lanzettensystem, was eine Hautperforation zur Erzeugung eines Blutstropfens und anschließend eine Analyse des Blutstropfens mit ein und demselben Messsystem ermöglicht.

Bei den beschriebenen, aus dem Stand der Technik bekannten Systemen ist jedoch das Problem der Feuchtigkeitsempfindlichkeit der Teststreifen bislang grundsätzlich nur unvollständig gelöst. Eine Einwirkung von Luftfeuchtigkeit, speziell bei erhöhter Temperatur und über längere Zeit, kann die Empfindlichkeit der Teststreifen beeinflussen und somit die Messung verfälschen. Um dies zu verhindern beinhalten beispielsweise die in WO 03/083469 A2 oder WO 02/055008 A2 gezeigten Systeme separate, austauschbare, luftdicht verschlossene Teststreifenmagazine, welche in das eigentliche Messgerät eingeführt werden. Auf diese Weise ist jedoch eine bauraumintensive Doppel-Umverpackung der Teststreifen erforderlich, da die Teststreifen nunmehr von der eigentlichen Magazinwand als Primärverpackung und zusätzlich von der Messgerätewand umgeben sind. Analog beschreibt auch WO 2006/047135 A1 einen komplexen Teststreifen-Dispenser mit einer Teststreifen-"Patrone". Hierbei umschließt ein Gerätegehäuse ein Magazin (Patrone), welches seinerseits eine äußere und eine innere Hülle aufweist. Die Teststreifen sind in dem Magazin feuchtigkeitsdicht gelagert. Da eine Optimierung der Baugröße für tragbare medizinische Messgeräte einen entscheidenden Faktor darstellt, ist der Nachteil der Doppelverpackung der aus dem Stand der Technik bekannten Systeme unter Umständen von entscheidender Bedeutung für die Akzeptanz des Messgerätes durch den Patienten.

Andere Systeme, wie beispielsweise das in US 5,489,414 gezeigte System, basieren auf individuell versiegelten Testelementen. Diese Testelemente können beispielsweise als Mehrfach-Testelemente mit individuell versiegelten Testfeldern ausgebildet sein. Dabei ergibt sich jedoch der Nachteil, dass vor Gebrauch die Versiegelung der Testelemente entfernt werden muss, was beispielsweise durch eine zusätzliche mechanische Aktion erfolgen muss oder durch eine manuelle Aktion des Patienten. Eine automatische Entfernung der Versiegelung, beispielsweise in Form eines Durchstoßens der Versiegelung, erfordert jedoch zusätzliche mechanische Elemente und Antriebselemente innerhalb des Messgeräts, welche wiederum den Bauraum und/oder den Energiebedarf der Systeme stark erhöhen.

Diese Beispiele zeigen, dass die Problematik, einerseits Einweg-Testelemente zum Nachfüllen der Messgeräte luftdicht verpackt beziehungsweise versiegelt zum Messgerät zu transportieren (beispielsweise über den Handel) und andererseits diese versiegelten Testelemente anschließend im Messgerät selbst unter Entfernung der Versiegelung einzusetzen, ein bislang nur unvollkommen gelöstes Problem darstellt.

### Aufgabe der Erfindung

Aufgabe der vorliegenden Erfindung ist es daher, ein tragbares Messsystem zur Analyse einer flüssigen Probe bereitzustellen, insbesondere für den Einsatz in der Medizintechnik, welches einen geringen Bauraum aufweist und dennoch eine möglichst luftdichte Aufbewahrung von Testelementen im Messgerät ermöglicht.

### Beschreibung der Erfindung

Diese Aufgabe wird durch die Erfindung mit den Merkmalen des unabhängigen Anspruchs gelöst. Vorteilhafte Weiterbildungen der Erfindung sind in den Unteransprüchen dargestellt.

Es wird ein tragbares Messsystem zur Analyse einer flüssigen Probe auf mindestens einen darin enthaltenen Analyten vorgeschlagen. Insbesondere kann es sich bei dem tragbaren Messsystem um ein System zur Glukosemessung, insbesondere Blutglukosemessung, und/oder zur Cholesterinmessung und/oder Koagulationsmessung handeln. Alternativ oder zusätzlich können jedoch auch andere Analyten ermittelt werden, beispielsweise deren Konzentration oder Anwesenheit, oder es können entsprechende andere Analysen durchgeführt werden, beispielsweise pH-Messungen oder ähnliche chemische Analysen. Auch können beispielsweise immunologische Messungen oder ähnliche Messungen mit der Testvorrichtung durchgeführt werden. Bei der Probe soll es sich insbesondere um eine flüssige Probe, beispielsweise Blut, Urin, Speichel oder Stuhl, handeln. Es sind jedoch auch andere Arten von Proben denkbar, beispielsweise gasförmige Proben.

Die Analyse beruht auf der Verwendung von Testelementen, beispielsweise optischen und/oder elektrochemischen Testelementen, wie sie aus dem Stand der Technik bekannt sind. Beispielsweise kann es sich bei diesen Testelementen um Teststreifen handeln.

Das tragbare Messsystem weist ein Messgerät mit einem feuchtigkeitsdichten Gehäuse mit einer Gehäuseinnenatmosphäre auf. Das feuchtigkeitsdichte Gehäuse verhindert ein Eindringen von Luftfeuchtigkeit und/oder Wasserdampf in die Gehäuseinnenatmosphäre im geschlossenen Zustand des feuchtigkeitsdichten Gehäuses. Dies kann beispielsweise durch entsprechende, aus dem Stand der Technik bekannte Dichtelemente und/oder Herstellungsverfahren bewerkstelligt werden.

Weiterhin weist das tragbare Messsystem mindestens einen in die Gehäuseinnenatmosphäre einführbaren Testelementträger auf, welcher mindestens eine Haltestruktur und mehrere von der mindestens einen Haltestruktur gehaltene Testelemente zur Analyse der Probenflüssigkeit aufweist. Der mindestens eine Testelementträger ist derart ausgestaltet, dass nach Einführen des Testelementträgers in die Gehäuseinnenatmosphäre die Testelemente der Gehäuseinnenatmosphäre ausgesetzt sind.

Insoweit weist das vorgeschlagene System Ähnlichkeiten beispielsweise mit dem in US 6,908,008 B2 dargestellten Magazinsystem auf. Im Gegensatz zu dem aus US 6,908,008 B2 bekannten System, bei welchem für die Applikation der flüssigen Probe ein Teststreifen aus dem luftdichten Magazin ausgegeben werden muss, besteht ein Grundgedanke der vorliegenden Erfindung darin, die Applikation der flüssigen Probe auf ein zu verwendendes Testelement innerhalb der Gehäuseinnenatmosphäre selbst vorzunehmen.

Zu diesem Zweck kann das vorgeschlagene tragbare Messsystem vorzugsweise einen Freigabemechanismus aufweisen, welcher ausgestaltet ist, um mindestens ein Testelement in eine Applikationsposition innerhalb der Gehäuseinnenatmosphäre zu befördern. In der Applikationsposition ist die flüssige Probe auf das mindestens eine Testelement aufbringbar.

Das vorgeschlagene tragbare Messsystem verzichtet somit auf eine luftdichte Primärverpackung um die Testelemente, welche vor der Applikation der flüssigen Probe entfernt werden müsste oder aus welcher die Testelemente vor Applikation der flüssigen Probe herausgenommen werden müssten. Auf diese Weise lässt sich der Bauraum des tragbaren Messsystems erheblich reduzieren.

Der mindestens eine Testelementträger kann beispielsweise in einer entfernbaren Verpackung, beispielsweise einer so genannten Blisterpackung, vertrieben und aufbewahrt werden. In dieser Verpackung sind die Testelemente gegen Luftfeuchtigkeit und andere atmosphärische Einflüsse geschützt. Zum Gebrauch öffnet der Patient die Verpackung und führt den Testelementträger in die Gehäuseinnenatmosphäre ein, zu welchem Zweck beispielsweise das feuchtigkeitsdichte Gehäuse aufgeklappt, aufgeschoben oder aufgeschraubt werden kann. Lediglich während dieser kurzen Einführzeit sind die Testelemente der Umgebungsluft ausgesetzt. Anschließend, nach Schließen des Gehäuses, sind die Testelemente ohne zusätzliche Versiegelung unmittelbar in der Gehäuseinnenatmosphäre gelagert. Die Abdichtung nach außen hin wird nun also vom Messgerät selbst übernommen. Zu diesem Zweck ist das Gehäuse des Messgeräts als ganzes oder teilweise feuchtigkeitsdicht ausgestaltet, in der Weise, dass die Gehäuseinnenatmosphäre geschützt ist.

Zusätzlich kann in der Gehäuseinnenatmosphäre ein Trockenmittel aufgenommen sein. Alternativ oder zusätzlich kann auch ein Trockenmittel mit dem mindestens einen Testelementträger verbunden sein, so dass mit jedem Austausch eines Testelementträgers ein neues, unverbrauchtes Trockenmittel in die Gehäuseinnenatmosphäre eingeführt wird. Die Menge des Trockenmittels ist dabei vorzugsweise so zu wählen, dass die durch die Diffusion durch die Gehäusewandungen und durch Einströmen durch Mikro-Undichtigkeiten über die gewünschte Lagerdauer eindringende Feuchtigkeit, sowie vorzugsweise zusätzlich die beim Applizieren der Probe in die Gehäuseinnenatmosphäre eingebrachte Feuchtigkeit, durch das Trockenmittel gebunden wird.

Nur während der kurzen Zeit, in der die flüssige Probe auf das mindestens eine zu verwendende Testelement aufgebracht wird, besteht die Möglichkeit, dass Luftfeuchtigkeit in die Gehäuseinnenatmosphäre eindringt. Zur Applikation der flüssigen Probe weist das Gehäuse eine Applikationsöffnung auf. Diese Applikationsöffnung ist mit mindestens einem Dichtelement ausgestattet. Dieses mindestens eine Dichtelement weist eine Dichtlippe, einen Dichtschieberund/oder eine Applikationsklappe, auf. Diese Applikationsöffnung und das damit verbundene mindestens eine Dichtelement werden grundsätzlich nur einmal pro Messsystem benötigt, so dass Bauraum und aufwändige Dichtungskonstruktionen für mehrere Öffnungen eingespart werden können.

Beispielsweise kann die Applikation der flüssigen Probe dadurch erfolgen, dass ein Patient, beispielsweise mittels eines Lanzettensystems, einen Blutstropfen auf der Hautoberfläche, beispielsweise einer Fingerkuppe, generiert, um anschließend durch Drücken oder Schieben mit der Fingerkuppe oder einem anderen Finger die Applikationsöffnung zu öffnen. Auf diese Weise kann der Blutstropfen dann innerhalb der Gehäuseinnenatmosphäre auf das mindestens eine Testelement aufgetragen werden.

Nach der Messung wird die Probe auf dem Testelement durch die trockene Umgebung in der Gehäuseinnenatmosphäre eingetrocknet. Zu diesem Zweck kann (s. o.) ein entsprechender Überschuss an Trockenmittel vorgesehen sein. So wird üblicherweise beispielsweise für die Trocknung einer flüssigen Probe pro 1 Mikroliter Probenvolumen jeweils eine Menge von ca. 10 mg eines Trockenmittels mit einer Kapazität von ca. 10 Gew.-% benötigt.

Um die Dichtungsfunktion des mindestens einen Dichtelements zu verbessern, kann weiterhin eine Druckausgleichsstruktur in dem tragbaren Messsystem vorgesehen sein. Diese Druckausgleichsstruktur verhindert, dass bei Änderung eines Drucks der Gehäuseinnenatmosphäre relativ zum Luftdruck außerhalb der Gehäuseinnenatmosphäre Wasserdampf und/oder Luftfeuchtigkeit in die Gehäuseinnenatmosphäre eindringen kann. Derartige Druckschwankungen ergeben sich beispielsweise bei einem Abkühlen oder Erwärmen des tragbaren Messsystems, welche mit einer Volumenänderung des Luftvolumens in der Gehäuseinnenatmosphäre und damit mit einer Druckschwankung verbunden sind. Diese Druckausgleichsstruktur kann beispielsweise ein flexibles Volumenänderungselement, insbesondere eine Druckausgleichsmembran, beinhalten. Auch andere Möglichkeiten sind denkbar.

Zur Überwachung der Gehäuseinnenatmosphäre kann weiterhin ein Feuchtesensor und/oder ein Temperatursensor vorgesehen sein. Mittels des Feuchtesensors lässt sich die Feuchte der Gehäuseinnenatmosphäre messen, und mittels des Temperatursensors die Temperatur. So lässt sich beispielsweise mittels eines Wamsystems ein Warnhinweis an einen Benutzer ausgeben, wenn die Feuchte der Gehäuseinnenatmosphäre und/oder die Temperatur der Gehäuseinnenatmosphäre einen (bzw. jeweils einen oder auch mehrere) vorgegebenen Schwellenwert übersteigt. Auch andere Arten von Messungen sind denkbar, beispielsweise eine Überwachung einer "Gesamtdosis" (beispielsweise ein zeitliches Integral der Feuchte und Temperatur), mit welcher die Testelemente in der Gehäuseinnenatmosphäre bislang beaufschlagt worden sind, um beispielsweise bei Erreichen einer Maximalbeaufschlagung eine Warnung an den Benutzer zu generieren, dass neue Testelemente verwendet werden sollten. So lässt sich beispielsweise ein "Klimaintegrator" überwachen, welcher beispielsweise ein zeitliches Intergral über Temperatur und Feuchte darstellt und entsprechend ein Warnhinweis bei Überschreiten eines Schwellenwertes generieren.

Die mindestens eine Haltestruktur kann auf verschiedene Weise ausgestaltet sein. Beispielsweise kann es sich dabei um eine Trommel mit umfangsseitigen Aufnahmeschlitzen für streifenförmige Testelemente handeln. Diese Trommel kann beispielsweise ein einfaches Spritzgussbauteil sein. Auch Testelement-Bänder mit mehreren Messbereichen sind einsetzbar, in welchem Fall beispielsweise ein Abspulmechanismus vorgesehen sein kann. Weiterhin kann die mindestens eine Haltestruktur beispielsweise mindestens einen in dem feuchtigkeitsdichten Gehäuse drehbaren Halterotor aufweisen.

Das tragbare Messsystem kann weiterhin eine elektronische Auswertungsvorrichtung zur Bestimmung der Anwesenheit und/oder Konzentration des mindestens einen Analyten aufweisen. Diese elektronische Auswertungsvorrichtung ist in diesem Falle vorzugsweise angepasst auf die Art und Funktionsweise des mindestens einen Testelements. So kann beispielsweise eine Auswertungsvorrichtung für elektrochemische Messungen mittels elektrochemischer Testelemente vorgesehen sein. Alternativ oder zusätzlich ist auch eine optische Auswertung denkbar.

Vorzugsweise weist das mindestens eine Testelement mindestens eine Reagenzschicht auf, welche ausgestaltet ist, um bei Kontakt mit dem mindestens einen nachzuweisenden Analyten mindestens eine Eigenschaft, insbesondere eine optische und/oder elektrochemische Eigenschaft, zu ändern. Dabei ist vorzugsweise bei in die Gehäuseinnenatmosphäre eingeführtem Testelementträger diese mindestens eine Reagenzschicht jedes einzelnen Testelements der Gehäuseinnenatmosphäre unmittelbar ausgesetzt. Unter "unmittelbar" kann dabei beispielsweise auch eine Verbindung der Reagenzschicht mit der Gehäuseinnenatmosphäre über eine Kapillare verstanden werden. Im Gegensatz zum Stand der Technik, wie beispielsweise in US 5,489,414, wird somit also auf eine Versiegelung, insbesondere eine individuelle Versiegelung, der Testelemente verzichtet.

Weiterhin ist es bevorzugt, wenn das tragbare Messsystem als solches bereits mindestens ein integriertes Lanzettensystem zur Perforation eines Hautbereichs aufweist. Vorzugsweise ist auch dieses integrierte Lanzettensystem in dem feuchtigkeitsdichten Gehäuse aufgenommen. Auf diese Weise kann über die Applikationsöffnung beispielsweise nacheinander, d. h. ohne Veränderung der Position der zu perforierenden Hautpartie, zunächst eine Perforation und Erzeugung eines Blutstropfens mit anschließender unmittelbarer Applikation dieses Blutstropfens auf ein Testelement erfolgen. So wird die Zahl der Öffnungsvorgänge des Gehäuses, bei welchen Luftfeuchtigkeit in die Gehäuseinnenatmosphäre eindringen könnte, minimiert. Weiterhin ist, wie oben bereits angeführt, grundsätzlich lediglich eine einzelne Applikationsöffnung erforderlich, welche abgedichtet werden muss. Auf diese Weise lässt sich der Bauraum des vorgeschlagenen tragbaren Messsystems sehr klein gestalten. Eine derartige Konstruktion ist mittels der aus dem Stand der Technik bekannten Magazinlösungen oder der Systeme, bei welchen die Testelemente einzeln versiegelt sind, nur schwer realisierbar. Das integrierte Lanzettensystem kann beispielsweise eine Mehrzahl von Einweglanzetten aufweisen, so dass für jede Perforation eine frische, unverbrauchte Lanzette verwendet werden kann.

Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktion einander entsprechende Elemente.

Im Einzelnen zeigt:
- Figur 1: ein dem Stand der Technik entsprechendes tragbares Messsystem mit doppelter Umhüllung von Testelementen;
- Figur 2: ein erfindungsgemäßes tragbares Messsystem ohne Primärverpackung der Testelemente;
- Figur 3: ein vollintegriertes tragbares Messsystem gemäß der Erfindung in Schnittdarstellung in Draufsicht; und
- Figur 4: das erfindungsgemäße tragbare Messsystem gemäß Figur 3 in Schnittdarstellung in Seitenansicht.

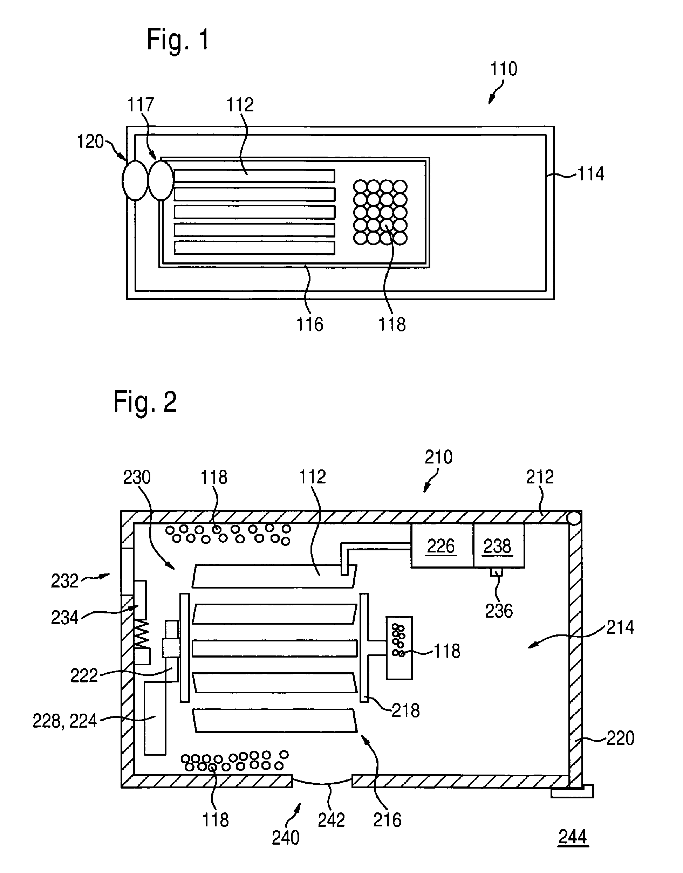

In Figur 1 ist ein dem Stand der Technik entsprechendes tragbares Messsystem 110 schematisch dargestellt, wie es beispielsweise dem in US 6,908,008 gezeigten System entspricht. In diesem Fall ist das tragbare Messsystem 110 als einfaches Aufbewahrungs- und Ausgabesystem (Dispenser) für Testelemente 112 in Form von Teststreifen ausgestaltet. Eine elektronische Auswertungsvorrichtung ist in diesem Beispiel nicht vorgesehen.

Das tragbare Messsystem 110 in Figur 1 weist ein Gehäuse 114 auf, welches in diesem Beispiel nicht notwendigerweise feuchtigkeitsdicht ausgestaltet ist. Stattdessen sind die Testelemente 112 in einer feuchtigkeitsdichten Primärverpackung 116 gelagert. In dieser feuchtigkeitsdichten Primärverpackung 116 ist weiterhin auch ein Trockenmittel 118 aufgenommen. Derartige Trockenmittel sind dem Fachmann bekannt. Beispielsweise kann es sich bei diesen Trockenmitteln um Silicagel, ein Molekularsieb und/oder andere, Luftfeuchtigkeit aufnehmende Chemikalien handeln.

Zum Gebrauch des tragbaren Messsystems gemäß Figur 1 wird die Primärverpackung 116 in das Gehäuse 114 eingeführt. Durch eine Ausgabeöffnung 117 der Primärverpackung 116 sowie eine Öffnung 120 des Gehäuses 114 kann mittels eines (hier nicht dargestellten) Ausgabemechanismus ein Testelement 112 ausgegeben werden. Um bei dieser Ausgabe ein Eindringen von Luftfeuchtigkeit in die Primärverpackung 116 zu verhindern, kann beispielsweise bei dieser Ausgabe eine individuelle Versiegelung (beispielsweise eine Siegelfolie) des Testelements 112 durchstoßen werden. Auch andere Ausgestaltungen sind bekannt, wie beispielsweise verschließbare Ausgabeschlitze oder ähnliches im Bereich der Ausgabeöffnung 117.

Nachteilig an dem bekannten tragbaren Messsystem 110 gemäß Figur 1 ist, wie oben dargestellt, der für die doppelte Umverpackung der Testelemente 112 erforderliche Bauraum.

Im Gegensatz zur Vorrichtung gemäß Figur 1 ist in Figur 2 ein erstes Ausführungsbeispiel eines erfindungsgemäßen tragbaren Messsystems 210 dargestellt. In diesem Ausfixhrungsbeispiel sei angenommen, dass das tragbare Messsystem 210 zur Ermittlung von Blutglukosekonzentrationen eingesetzt wird. Wie oben dargestellt, sind jedoch auch andere Anwendungsmöglichkeiten denkbar. Zu diesem Zweck weist in diesem Beispiel das tragbare Messsystem 210 ein feuchtigkeitsdichtes Gehäuse 212 auf, welches eine Gehäuseinnenatmosphäre 214 umschließt.

Mit "Gehäuseinnenatmosphäre" ist dabei gleichzeitig der vom feuchtigkeitsdichten Gehäuse 212 umschlossene Hohlraum sowie auch die Atmosphäre, also die Eigenschaften des Gases (in der Regel Luft) in dem Hohlraum bezeichnet. In der vereinfachten Darstellung gemäß Figur 2 weist das feuchtigkeitsdichte Gehäuse 212 einen einfachen Hohlraum auf, wobei jedoch auch andere Ausgestaltungen denkbar sind. So ist es beispielsweise nicht unbedingt erforderlich, dass das feuchtigkeitsdichte Gehäuse als Ganzes feuchtigkeitsdicht ausgestaltet ist. Gefordert wird lediglich, dass das feuchtigkeitsdichte Gehäuse 212 den Bereich, in welchem Testelemente 112 aufgenommen sind (Gehäuseinnenatmosphäre 214) feuchtigkeitsdicht umschließt.

Unter "feuchtigkeitsdicht" wird dabei nicht nur ein Schutz gegen Spritzwasser oder Regen, sondern auch ein Schutz gegenüber Wasserdampf und Luftfeuchtigkeit verstanden. Auch eine vollständige Wasserdichtigkeit kann vorgesehen sein. Dabei ist jedoch zu beachten, dass ein hundertprozentiger Schutz gegenüber Luftfeuchtigkeit technisch zumeist kaum realisierbar ist, so dass unter "feuchtigkeitsdicht" auch verstanden werden soll, dass das Eindringen von Luftfeuchtigkeit lediglich verlangsamt wird. Die diffusionsbedingt durch die Wände des Gehäuses 114 eindringende Feuchtigkeit wird durch das Trockenmittel 118 gebunden. Dringt beispielsweise 1 mg Luftfeuchtigkeit pro Tag ein, so gewährt ein Vorrat an Trockenmittel 118 von 1 g bei 10 Gew.-% Bindefähigkeit eine Aufbewahrungsfähigkeit von ca. 100 Tagen.

Das tragbare Messsystem 210 gemäß dem Ausführungsbeispiel in Figur 1 basiert auf einer Messung mittels Testelementen 112, welche gemäß dem Stand der Technik ausgebildet sein können. Beispielsweise kann es sich dabei um elektrochemische Teststreifen, beispielsweise Kapillarspalt-Testelemente, und/oder um optische Teststreifen handeln.

Die Testelemente 112 sind auf einem in die Gehäuseinnenatmosphäre 214 einführbaren Testelementträger 216 aufgenommen, welcher wiederum eine Haltestruktur 218 aufweist. Diese Haltestruktur 218 kann, wie in Figur 2, beispielsweise in Form einer Trommel ausgestaltet sein, auf welcher die Testelemente 112 umfangsseitig in radialen Längsschlitzen aufgenommen sind. Dabei wird, im Gegensatz zu Figur 1, auf eine Primärverpackung 116 verzichtet, was bedeutet, dass die Testelemente 112 unmittelbar der Gehäuseinnenatmosphäre 214 ausgesetzt sind. Das feuchtigkeitsdichte Gehäuse 212 ersetzt somit die Primärverpackung 116 und nimmt damit die Funktionen der Elemente 114 und 116 gemäß Figur 1 gleichzeitig wahr.

Der Testelementträger 216 kann über eine feuchtigkeitsdicht verschließbare Einführöffnung 220 (beispielsweise eine durch einen O-Ring abgedichtete, verriegelbare Klappe) dem feuchtigkeitsdichten Gehäuse 212 entnommen und gegen einen mit unverbrauchten Testelementen 112 bestückten Testelementträger 216 ausgetauscht werden, welcher zu diesem Zweck beispielsweise einer Blisterpackung entnommen wird. Der Testelementträger 216 rastet beim Einführen in einer Aufnahme 222 ein, in welcher er mittels eines Antriebs 224 drehbar gelagert ist.

Weiterhin ist in der Gehäuseinnenatmosphäre ein Trockenmittel 118 aufgenommen. Alternativ oder zusätzlich kann auch Trockenmittel 118 auf und/oder in dem Testelementträger 216 aufgenommen sein, so dass dieses Trockenmittel 118 mit jedem Austausch eines Testelementträgers 216 ebenfalls erneuert wird.

Der Antrieb 224 wirkt in diesem Beispiel als Freigabemechanismus 228. Mittels dieses Freigabemechanismus 228 wird ein zu verwendendes Testelement 112 in eine Applikationsposition 230 befördert. In dieser Applikationsposition 230 kann eine flüssige Probe über eine Applikationsöffnung 232 im feuchtigkeitsdichten Gehäuse 212 auf das Testelement 112 aufgebracht werden. Zu diesem Zweck weist die Applikationsöffnung 232 ein Dichtelement 234 auf. Dieses Dichtelement 234 ist in diesem Ausführungsbeispiel gemäß Figur 2 als Dichtschieber ausgebildet, welcher beispielsweise mit einem Finger des Patienten beiseite geschoben werden kann, so dass die Applikationsöffnung 232 freigeben wird. Ein auf dem Finger befindlicher Blutstropfen kann dann auf das zu verwendende Testelement 112 in der Applikationsposition 230 aufgebracht werden. Zu diesem Zweck kann auch das zu verwendende Testelement 112 durch einen (hier nicht dargestellten) Mechanismus näher in die Nähe der Applikationsöffnung 232 geschoben werden.

In dem Ausführungsbeispiel gemäß Figur 2 sei im Folgenden angenommen, dass es sich bei den Testelementen 112 um elektrochemische Testelemente, beispielsweise um Testelemente in Form von Kapillarspalt-Testelementen, handelt. Dementsprechend ist eine elektronische Auswertungsvorrichtung 226 vorgesehen, welche das in der Applikationsposition 230 befindliche Testelement 112 elektrisch kontaktiert und einen entsprechenden Messwert generiert. Die elektronische Auswertungsvorrichtung 226 kann zu diesem Zweck beispielsweise einen Mikrocomputer, eine Auswertungselektronik, Bedienelemente (z. B. Druckknöpfe, Schalter, Taster etc.), akustische und/oder optische Ausgabeelemente (z. B. ein oder mehrere Displays), Datenspeicher sowie weitere Elemente umfassen. Derartige elektronische Auswertungsvorrichtungen 226 sind aus dem Stand der Technik bekannt. Die elektronische Auswertungsvorrichtung 226 kann beispielsweise auch den Freigabemechanismus 228 steuern.

Weiterhin sind in dem Ausführungsbeispiel gemäß Figur 2 in der Gehäuseinnenatmosphäre 214 ein Feuchtesensor 236 (wobei es sich alternativ oder zusätzlich auch um einen Temperatursensor handeln kann) und ein Warnsystem 238 vorgesehen. Beispielsweise kann das Warnsystem 238 Bestandteil der elektronischen Auswertungsvorrichtung 226 sein. Das Warnsystem kann beispielsweise derart ausgestaltet sein, dass dieses eine akustische und/oder optische Warnung an einen Benutzer ausgibt, sobald eine Luftfeuchtigkeit innerhalb der Gehäuseinnenatmosphäre 214 einen vorgegebenen Schwellwert übersteigt. Wie oben beschrieben, ist auch eine andere Funktionalität denkbar, beispielsweise eine Integration der Luftfeuchtigkeit über die Zeit. Diese kann auch unter Berücksichtigung einer gemessenen Temperatur in der Gehäuseinnenatmosphäre 214 erfolgen.

Das feuchtigkeitsdichte Gehäuse 214 ist beispielsweise als ein- oder mehrteiliges Spritzgussbauteil ausgebildet. Verfahren, um derartige Spritzgussgehäuse feuchtigkeitsdicht auszugestalten, sind dem Fachmann bekannt. Zu diesem Zweck können beispielsweise entsprechende Kunststoffe mit geringer Permeabilität für Luftfeuchtigkeit und Sauerstoff und/oder entsprechende Dichtvorrichtungen (z. B. Dichtringe) verwendet werden. Dementsprechend kann auch das Dichtelement 234 auf verschiedene Weisen gestaltet sein. Wie oben beschrieben, können somit alternativ oder zusätzlich zu dem in Figur 2 dargestellten, federgelagerten Dichtschieber beispielsweise auch eine oder mehrere Dichtlippen verwendet werden. Derartige Dichtelemente sind beispielsweise mittels eines Mehrkomponenten-Spritzgusses und/oder durch Umspritzen entsprechender Dichtelemente (beispielsweise Gummilippen) in einem geeigneten Spritzgusswerkzeug herstellbar. Das bzw. die Dichtelemente können (alternativ oder kumulativ) entweder vom Patienten selbst geöffnet werden (z. B. über manuelle Schieber etc.), oder, selbsttätig, durch einen eigenen Öffnungs-Antrieb.

Weiterhin weist das feuchtigkeitsdichte Gehäuse 212 in dem Ausführungsbeispiel gemäß Figur 2 eine Druckausgleichsstruktur 240 auf. Diese Druckausgleichsstruktur 240 ist in diesem Ausführungsbeispiel als Druckausgleichsmembran 242 ausgebildet, welche in das feuchtigkeitsdichte Gehäuse 212 integriert ist (beispielsweise wiederum durch Umspritzen). Diese Druckausgleichsmembran 242 verhindert zum einen ein Eindringen von Luftfeuchtigkeit in die Gehäuseinnenatmosphäre 214 und ermöglicht zum anderen einen Druckausgleich zwischen der Gehäuseinnenatmosphäre 214 und der äußeren Atmosphäre 244. Dadurch wird ein "Atmen" des tragbaren Messsystems 210 verhindert, bei welchem beispielsweise infolge von Temperaturschwankungen ein Luftaustausch durch die Applikationsöffnung 232 erfolgen könnte.

Insgesamt kann das feuchtigkeitsdichte Gehäuse 212 somit derart ausgestaltet sein, dass Luftfeuchtigkeit im Wesentlichen von der Gehäuseinnenatmosphäre 214 ferngehalten wird. Das feuchtigkeitsdichte Gehäuse 212 kann zudem Spritzwasserschutz bis hin zur Wasserdichtigkeit des gesamten tragbaren Messsystems 210 gewährleisten. Auch eine Reinigung des tragbaren Messsystems 210 mit aggressiven Reinigungsmitteln wird dadurch weitgehend ermöglicht. Die durch Applikation der flüssigen Probe über die Applikationsöffnung 232 in die Gehäuseinnenatmosphäre 214 eingeführte Feuchtigkeit kann in der Regel durch das Trockenmittel 118 problemlos aufgenommen werden.

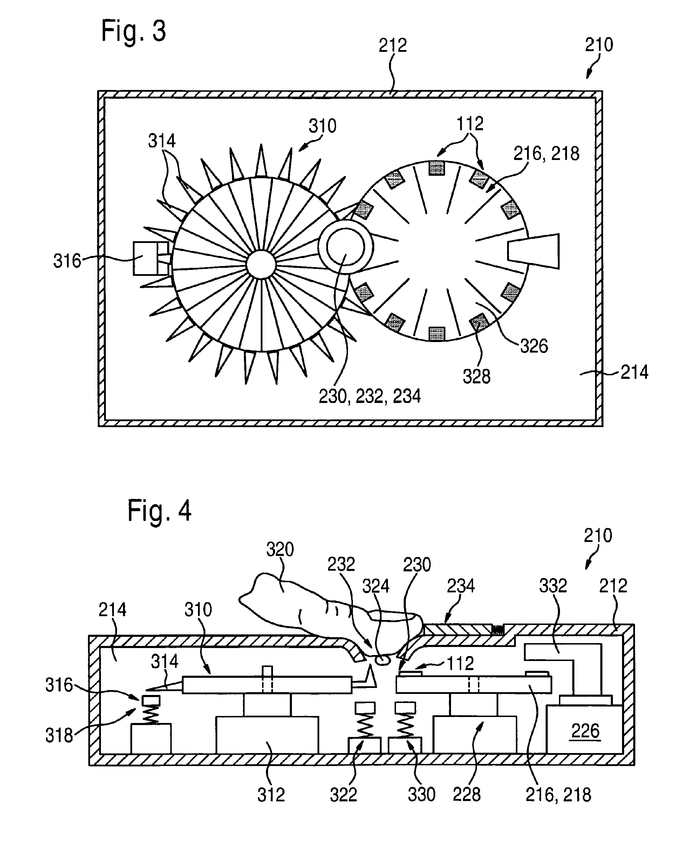

Anstelle des in Figur 2 beispielhaft dargestellten Testelementträgers 216 mit der trommelförmigen Haltestruktur 218 können problemlos auch andere Strukturen eingesetzt werden, beispielsweise Stapel-, Scheiben-, Reihen- und/oder Zickzack-Anordnungen. In den Figuren 2 und 3 ist ein weiteres Beispiel dargestellt, in welchem der Testelementträger 216 kreisscheibenförmig in Form eines Teststreifenrades ausgestaltet ist.

Das tragbare Messsystem 210 gemäß dem Ausführungsbeispiel in den Figuren 2 und 3 beinhaltet weiterhin ein integriertes Lanzettensystem 310 in Form eines durch einen Antrieb 312 drehbar gelagerten Multispitzen-Lanzettenrades.

Im Betrieb wird zunächst eine zu verwendende Lanzette 314 in eine Knickstation 316 gedreht. In dieser Knickstation 316 wird mittels eines Federmechanismus 318 die zu verwendende Lanzette 314 um 90° nach oben (in Figur 3) geknickt. Anschließend wird mittels des Antriebs 312 das Lanzettenrad um 180° gedreht, bis die nach oben geknickte Lanzette unterhalb der Applikationsöffnung 232 im Gehäuse 212 steht. Die Applikationsöffnung 232 ist in diesem Ausführungsbeispiel als Konus ausgeführt und ist wiederum mit einem Dichtelement 234 verschlossen. Das Dichtelement 234 kann mit dem Finger 320 des Patienten beiseite geschoben und dadurch die Applikationsöffnung 232 freigelegt werden. Der Finger 320 verschließt diese Applikationsöffnung 232 dabei gleichzeitig. Durch Aufpressen des Fingers 320 auf die Applikationsöffnung 232 bildet die Hautpartie des Fingers 320 im Bereich der Applikationsöffnung 232 eine ins Innere des Gehäuses 212 gewölbte Beule. Mittels eines Lanzetten-Federmechanismus 322 wird beim Auslösen des tragbaren Messsystems 210 die sich unterhalb der Applikationsöffnung 232 befindliche Lanzette 314, welche nach oben gebogen ist, beschleunigt und perforiert die Hautpartie des Fingers 320 innerhalb der Applikationsöffnung 232. Dadurch bildet sich ein Blutstropfen 324.

Der in diesem Ausführungsbeispiel gemäß den Figuren 3 und 4 kreisscheibenförmig ausgebildete Testelementträger 216 ist in Kreisscheiben-Sektoren 326 unterteilt. Jeder dieser Kreisscheiben-Sektoren 326 bildet ein Testelement 112. Jedes dieser Testelemente 112 weist eine Reagenzschicht 328 auf, welche, wie oben beschrieben, auf die Anwesenheit und/oder Konzentration des Analyten in der flüssigen Probe (Blutstropfen 324) reagiert. In diesem Ausführungsbeispiel gemäß den Figuren 3 und 4 handelt es sich bei dieser Reagenzschicht 328 beispielsweise um eine bei Anwesenheit von Glukose eine Farbreaktion durchführende Reagenzschicht 328, d. h. eine Reagenzschicht 328, welche durch Reaktion mit Glukose ihre Farbe und/oder ihre Fluoreszenzeigenschaften ändert.

Nach Perforation der Hautpartie im Bereich der Applikationsöffnung 232 wird nun der Testelementträger 216 durch einen Testelement-Federmechanismus 330 im Bereich der unterhalb der Applikationsöffnung 232 befindlichen Applikationsposition kurzfristig nach oben, hin zur Applikationsöffnung 232, gebogen. Dadurch wird der Blutstropfen 324 auf die Reagenzschicht 328 des sich in der Applikationsposition 230 befindlichen Testelements 112 aufgebracht, und es kann die beschriebene Reaktion erfolgen.

Anschließend wird mittels des Freigabemechanismus 228 der Testelementträger 216 um 180° gedreht, so dass das Testelement 112, auf welches der Blutstropfen 324 appliziert wurde, ober- oder unterhalb eines optischen Auslesers 332 platziert ist. Dieser optische Ausleser 332 führt beispielsweise eine einfache optische Messung oder eine Fluoreszenzanregungs-Messung durch. Der optische Ausleser 332 ist mit der elektronischen Auswertungsvorrichtung 226 verbunden, welche analog zu Figur 2 ausgestaltet sein kann und beispielsweise einen Mikroprozessor, Bedienelemente, Displays, Datenspeicher oder ähnliches umfassen kann.

Das vollintegrierte tragbare Messsystem 210 gemäß dem Ausführungsbeispiel in den Figuren 3 und 4 zeigt besonders deutlich die Vorteile der erfindungsgemäßen Lösung. Erfindungsgemäß ist die Reagenzschicht 328 hier unmittelbar der Gehäuseinnenatmosphäre 214 ausgesetzt. Eine separate Versiegelung der einzelnen Testelemente 112 ist weder erforderlich noch zweckmäßig. Müsste zunächst eine Versiegelung von den einzelnen Reagenzschichten 328 entfernt werden, so würde dies einen zusätzlichen mechanischen Aufwand erfordern, welcher sich wiederum nachteilig auf den Energiebedarf sowie auf den Bauraum auswirken würde, da weitere mechanische Vorrichtungen und Betätigungselemente erforderlich wären. Wiederum kann beispielsweise ein (in Figur 3 und in Figur 4 nicht dargestelltes) Trockenmittel 118 in den hier kreisscheibenförmig ausgestalteten Testelementträger 216 integriert sein.

### Bezugszeichenliste

- 110: tragbares Messsystem (Stand der Technik)
- 112: Testelement
- 114: Gehäuse
- 116: Primärverpackung
- 117: Ausgabeöffnung
- 118: Trockenmittel
- 120: Öffnung

- 210: tragbares Messsystem
- 212: feuchtigkeitsdichtes Gehäuse
- 214: Gehäuseinnenatmosphäre
- 216: Testelementträger
- 218: Haltestruktur
- 220: Einführöffnung
- 222: Aufnahme
- 224: Antrieb
- 226: elektronische Auswertungsvorrichtung
- 228: Freigabemechanismus
- 230: Applikationsposition
- 232: Applikationsöffnung
- 234: Dichtelement
- 236: Feuchtesensor
- 238: Warnsystem
- 240: Druckausgleichsstruktur
- 242: Druckausgleichsmembran
- 244: äußere Atmosphäre

- 310: integriertes Lanzettensystem
- 312: Antrieb
- 314: Lanzette
- 316: Knickstation
- 318: Federmechanismus
- 320: Finger
- 322: Lanzettenfedermechanismus
- 324: Blutstropfen
- 326: Kreisscheiben-Sektoren
- 328: Reagenzschicht
- 330: Testelement-Federmechanismus
- 332: optischer Ausleser

## Patentansprüche

1. Tragbares Messsystem (210) zur Analyse einer flüssigen Probe auf mindestens einen darin enthaltenen Analysen, aufweisend
- ein feuchtigkeitsüzchtes Gehäuse (212) mit einer Gehäuseinnenatmosphärre (214), wobei das feuchtigkeitsdichte Gehäuse (212) ein Eindringen von Luftfeuchtigkeit und/oder Wasserdampf in die Gehäuseinnenatmosphäre (214) verhindert, und
- mindestens einen in die Gehäuseinnenatmosphäre (214) einführbaren Testelementträger (216), wobei der mindestens eine Testelementträger (216) mindestens eine Haltestruktur (218) und mehrere von der mindestens einen Haltestruktur (218) gehaltene Testelemente (112) zur Analyse der flüssigen Probe aufweist, wobei der mindestens eine Testelementträger (216) derart ausgestaltet ist, dass nach Einführen in die Gehäuseinnenatmosphäre (214) die Testelemente (112) der Gehäuseinnenatmosphäre (214) ausgesetzt sind,
**dadurch gekennzeichnet, dass** die flüssige Probe innerhalb der Gehäuseinnenatmosphäre (214) auf die Testelemente (112) aufbringbar ist, wobei mindestens eine Applikationsöffnung (232) vorgesehen ist, welche mindestens eines der folgenden Dichtelemente (234) aufweist: eine Dichtlippe, einen Dichtschieber, eine Applikationsklappe.

2. Tragbares Messsystem (210) gemäß dem vorhergehenden Anspruch, **gekennzeichnet durch** einen Freigabemechanismus (228), wobei der Freigabemechanismus (228) ausgestaltet ist, um die Testelemente (112) in eine Applikationsposition (230) innerhalb der Gehäuseinnenatmosphäre (214) zu befördern, wobei in der Applikationsposition (230) die flüssige Probe auf die Testelemente (112) aufbringbar ist.

3. Tragbares Messsystem (210) gemäß einem der beiden vorhergehenden Ansprüche, **gekennzeichnet durch** ein in der Gehäuseinnenatmosphäre (214) aufgenommenes Trockenmittel (118).

4. Tragbares Messsystem (210) gemäß einem der vorhergehenden Ansprüche, **gekennzeichnet durch** eine Druckausgleichsstruktur (240) zur Verhinderung von Eindringen von Wasserdampf und/oder Luftfeuchtigkeit bei Änderung eines Drucks der Gehäuseinnenatmosphäre (214) relativ zum Luftdruck außerhalb der Gehäuseinnenatmosphäre (214).

5. Tragbares Messsystem (210) gemäß dem vorhergehenden Anspruch, **gekennzeichnet durch** ein flexibles Volumenänderungselement, insbesondere eine Druckausgleichsmembran (242).

6. Tragbares Messsystem (210) gemäß einem der vorhergehenden Ansprüche, **gekennzeichnet durch** einen Feuchtesensor (236) und/oder einen Temperatursensor zur Messung einer Feuchte und/oder Temperatur der Gehäuseinnenatmosphäre (214).

7. Tragbares Messsystem (210) gemäß dem vorhergehenden Anspruch, **gekennzeichnet durch** ein Warnsystem (238) zur Ausgabe eines Warnhinweises an einen Benutzer, wenn mindestens einer der folgenden Werte einen oder mehrere vorgegebene Schwellenwerte übersteigt: die Feuchte der Gehauseüanenatmosphäre (214); die Temperatur der Gehäuseinnenatmosphäre (214); ein Klimaintegrator, welcher Zeit, Feuchte und Temperatur berücksichtigt.

8. Tragbares Messsystem (210) gemäß einen der vorhergehenden Ansprüche, **dadurch gekennzeichnet, dass** die mindestens eine Haltestruktur (218) mindestens einen in dem feuchtigkeitsdichten Gehäuse (212) drehbaren Halterotor und/oder eine Trommel aufweist

9. Tragbares Messsystem (210) gemäß einem der vorhergehenden Ansprüche, **gekennzeichnet durch** ein mit dem Testelementträger (216) verbundenes Trockenmittel (118).

10. Tragbares Messsystem (210) gemäß einem der vorhergehenden Ansprüche, **gekennzeichnet durch** eine elektronische Auswertungsvorrichtung (226) zur Bestimmung der Anwesenheit und/oder der Konzentration des mindestens einen Analyten.

11. Tragbares Messsystem (210) gemäß einem der vorhergehenden Ansprüche, **gekennzeichnet durch** mindestens ein integriertes Lanzettensystem (310) zur Perforation eines Hautbereiches.

12. Tragbares Messsystem (210) gemäß dem vorhergehenden Anspruch, **dadurch gekennzeichnet, dass** das mindestens eine integrierte Lanzettensystem (310) in dem feuchtigkeitsdichten Gehäuse (212) angenommen ist.

13. Tragbares Messsystem (210) gemäß einem der beiden vorhergehenden Ansprüche, **dadurch gekennzeichnet, dass** das mindestens eine integrierte Lanzettensystem (310) eine Mehrzahl von als Einweglanzetten ausgebildeten Lanzetten (314) aufweist.

14. Tragbares Messsystem (210) gemäß einem der vorhergehenden Ansprüche, **dadurch gekennzeichnet, dass** die Testelemente (112) mindestens eine Reagenzschicht (328) aufweisen, wobei die mindestens eine Reagenzschicht (328) ausgestaltet ist, um bei Kontakt mit dem mindestens einen nachzuweisenden Analyten mindestens eine Eigenschaft, insbesondere eine optische und/oder elektrochemische Eigenschaft, zu ändern, wobei bei in die Gehäuseinnenatmosphäre (214) eingeführtem Testelementträger (216) die mindestens eine Reagenzschicht (328) jedes Testelement (112) der Gehäuseinnenatmosphäre (214) ausgesetzt ist.

## Claims

1. Portable measuring system (210) which is used to analyze a liquid sample for at least one analyte contained therein, comprising
- a moisture-proof housing (212) with an internal atmosphere (214), said moisture-proof housing (212) preventing air moisture and/or water vapor from penetrating into the internal atmosphere (214) of the housing, and
- at least one test element support (216) which can be inserted into the internal atmosphere (214) of the housing, the at least one test element support (216) having at least one retaining structure (218) and a plurality of test elements (112) that are supported by the at least one retaining structure (218) and that are used to analyze the liquid sample, the at least one test element support (216) being designed in such a way that, after insertion into the internal atmosphere (214) of the housing, the test elements (112) are exposed to said internal atmosphere (214),
**characterized in that** the liquid sample can be applied to the test elements (112) within the internal atmosphere (214) of the housing, and at least one application opening (232) is provided which has at least one of the following sealing elements (234): a sealing lip, a sealing slide, an application flap.

2. Portable measuring system (210) according to the preceding claim, **characterized by** a release mechanism (228), said release mechanism (228) being designed to convey the test elements (112) into an application position (230) within the internal atmosphere (214) of the housing, the liquid sample being able to be applied to the test elements (112) in the application position (230).

3. Portable measuring system (210) according to one of the preceding two claims, **characterized by** a desiccant (118) received in the internal atmosphere (214) of the housing.

4. Portable measuring system (210) according to one of the preceding claims, **characterized by** a pressure-equalizing structure (240) for preventing penetration of water vapor and/or moisture when a pressure of the internal atmosphere (214) of the housing changes relative to the air pressure outside the internal atmosphere (214) of the housing.

5. Portable measuring system (210) according to the preceding claim, **characterized by** a flexible volume-modifying element, in particular a pressure-equalizing membrane (242).

6. Portable measuring system (210) according to one of the preceding claims, **characterized by** a moisture sensor (236) and/or a temperature sensor for measuring the moisture and/or temperature of the internal atmosphere (214) of the housing.

7. Portable measuring system (210) according to the preceding claim, **characterized by** a warning system (238) for emitting a warning to a user if at least one of the following values exceeds one or more predefined threshold values: the moisture of the internal atmosphere (214) of the housing; the temperature of the internal atmosphere (214) of the housing; a climate integrator that takes account of time, moisture and temperature.

8. Portable measuring system (210) according to one of the preceding claims, **characterized in that** the at least one retaining structure (218) has at least one retaining rotor and/or drum that is able to rotate in the moisture-proof housing (212).

9. Portable measuring system (210) according to one of the preceding claims, **characterized by** a desiccant (118) connected to the test element support (216).

10. Portable measuring system (210) according to one of the preceding claims, **characterized by** an electronic evaluation device (226) for determining the presence and/or the concentration of the at least one analyte.

11. Portable measuring system (210) according to one of the preceding claims, **characterized by** at least one integrated lancet system (310) for perforating an area of skin.

12. Portable measuring system (210) according to the preceding claim, **characterized in that** the at least one integrated lancet system (310) is received in the moisture-proof housing (212).

13. Portable measuring system (210) according to one of the preceding two claims, **characterized in that** the at least one integrated lancet system (310) has a plurality of lancets (314) designed as disposable lancets.

14. Portable measuring system (210) according to one of the preceding claims, **characterized in that** the test elements (112) have at least one reagent layer (328), said at least one reagent layer (328) being designed to change at least one property, in particular an optical and/or electro-chemical property, upon contact with the at least one analyte to be detected, and, with the test element support (216) inserted into the internal atmosphere (214) of the housing, the at least one reagent layer (328) of each test element (112) is exposed to the internal atmosphere (214) of the housing.

## Revendications

1. Système portable de mesure (210) destiné à analyser au moins un analyte que contient un échantillon liquide, le système présentant
- un boîtier (212) étanche à l'humidité doté d'une atmosphère intérieure (214), le boîtier (212) étanche à l'humidité empêchant la pénétration de l'humidité de l'air et/ou de vapeur d'eau dans l'atmosphère intérieure (214) du boîtier et
- au moins un support (216) d'élément de test qui peut être inséré dans l'atmosphère intérieure (214) du boîtier, le ou les supports (216) d'élément de test présentant au moins une structure de maintien (218) et plusieurs éléments de test (112) maintenus par une structure de maintien (218) pour l'analyse de l'échantillon liquide, le ou les supports (216) d'élément de test étant configurés de telle sorte que les éléments de test (112) soient exposés à l'atmosphère intérieure (214) du boîtier après avoir été insérés dans l'atmosphère intérieure (214) du boîtier,
**caractérisé en ce que**
l'échantillon liquide peut être appliqué sur les éléments de test (112) à l'intérieur de l'atmosphère intérieure (214) du boîtier et
**en ce qu'**au moins une ouverture d'application (232) qui présente au moins l'un des éléments d'étanchéité (234) suivants : une lèvre d'étanchéité, un coulisseau d'étanchéité ou un clapet d'application est prévue.

2. Système portable de mesure (210) selon la revendication précédente, **caractérisé par** un mécanisme de libération (228), le mécanisme de libération (228) étant configuré pour transporter les éléments de test (112) dans une position d'application (230) à l'intérieur de l'atmosphère intérieure (214) du boîtier, l'échantillon liquide pouvant être appliqué sur les éléments de test (112) dans la position d'application (230).

3. Système portable de mesure (210) selon l'une des deux revendications qui précèdent **caractérisé par** un moyen de séchage (118) logé dans l'atmosphère intérieure (214) du boîtier.

4. Système portable de mesure (210) selon l'une des revendications précédentes, **caractérisé par** une structure (240) d'équilibrage de pression qui empêche la pénétration de vapeur d'eau et/ou d'humidité de l'air en cas de modification de la pression de l'atmosphère intérieure (214) du boîtier par rapport à la pression atmosphérique qui règne à l'extérieur de l'atmosphère intérieure (214) du boîtier.

5. Système portable de mesure (210) selon la revendication précédente, **caractérisé par** un élément flexible de modification de volume, en particulier une membrane (242) d'équilibrage de pression.

6. Système portable de mesure (210) selon l'une des revendications précédentes, **caractérisé par** un détecteur d'humidité (236) et/ou par une sonde de température qui mesure l'humidité et/ou la température de l'atmosphère intérieure (214) du boîtier.

7. Système portable de mesure (210) selon la revendication précédente, **caractérisé par** un système d'avertissement (238) qui délivre une indication d'avertissement à l'utilisateur lorsqu'au moins l'une des valeurs suivantes dépasse une ou plusieurs valeurs de seuil prédéterminées : l'humidité de l'atmosphère intérieure (214) du boîtier, la température de l'atmosphère intérieure (214) du boîtier ou un intégrateur de climat qui tient compte du temps, de l'humidité et de la température.

8. Système portable de mesure (210) selon l'une des revendications précédentes, **caractérisé en ce que** la ou les structures de maintien (218) présentent au moins un rotor de maintien et/ou un tambour qui peuvent tourner dans le boîtier (212) étanche à l'humidité.

9. Système portable de mesure (210) selon l'une des revendications précédentes, **caractérisé par** un agent de séchage (118) relié au support (216) d'élément de test.

10. Système portable de mesure (210) selon l'une des revendications précédentes, **caractérisé par** un dispositif électronique d'évaluation (226) qui détermine la présence et/ou la concentration du ou des analytes.

11. Système portable de mesure (210) selon l'une des revendications précédentes, **caractérisé par** au moins un système intégré de lancette (310) qui perfore une zone de la peau.

12. Système portable de mesure (210) selon la revendication précédente, **caractérisé en ce que** le ou les systèmes intégrés de lancette (310) sont logés dans le boîtier (212) étanche à l'humidité.

13. Système portable de mesure (210) selon l'une des deux revendications qui précèdent, **caractérisé en ce que** le ou les systèmes intégrés de lancette (310) présentent plusieurs lancettes (314) configurées comme lancettes à usage unique.

14. Système portable de mesure (210) selon l'une des revendications précédentes, **caractérisé en ce que** les éléments de test (112) présentent au moins une couche (328) de réactif, la ou les couches (328) de réactif étant configurées pour modifier au moins une propriété et en particulier une propriété optique et/ou électrochimique au contact avec le ou les analytes à détecter et **en ce que** lorsque le support (216) d'élément de test est inséré dans l'atmosphère intérieure (214) du boîtier, la ou les couches (328) de réactif de chaque élément de test (112) sont exposées à l'atmosphère intérieure (214) du boîtier.
